# EUROPEAN PATENT APPLICATION

(11) **EP 3 259 990 A1**
(43) Date of publication of application: **27.12.2017**
(21) Application number: 16175349.6
(22) Date of filing: 20.06.2016
(51) Int. Cl.: A01N 33/04, A01P 5/00, A01P 7/02, A01P 7/04, A61K 9/00

(54) **METHODS FOR CONTROLLING ECTOPARASITES IN NON-HUMAN MAMMALS**

(71) Applicant: Ceva Santé Animale, 33500 Libourne Cedex (FR)
(72) Inventor: The designation of the inventor has not yet been filed
(74) Representative: Cabinet Becker et Associés

(57) **Abstract**

The present invention relates to novel methods of treatment in the field of veterinary medicine, particularly methods for controlling ectoparasites in non-human mammals.

## Description

### FIELD OF THE INVENTION

The present invention relates to novel methods of treatment in the field of veterinary medicine, particularly methods for controlling ectoparasites in non-human mammals.

### BACKGROUND OF THE INVENTION

Non-human mammals, particularly domestic animals or pets such as dogs or cats, are often susceptible to parasites infestations and may serve as hosts for a large number of ectoparasites. In view of the increasing of domestic animals in the society, the control of parasites and treatment of parasites infestations has been one of the primary objectives of veterinary pharmaceutical companies, not only because of the discomfort of said infestations, but also because of the possible transmission of said infestations to other animals and human beings. In this context, compositions comprising one or several active ingredients as insecticides have been developed for treating parasites infestations and/or controlling parasites.
Washability of topical parasiticides by shampoos is often pointed as a weakness and it is generally recommended to wait for at least 48h between shampoo and topical treatment. Therefore, there is a need to provide shampoos that can still be used during a treatment against parasites without impairing said treatment.

### SUMMARY OF THE INVENTION

In that context, the inventors have discovered surprisingly that a shampoo comprising a specific compound can be used even while performing a treatment for parasites infestations and/or controlling parasites and does have a negative impact on the efficiency of a topical treatment against ectoparasites.

The present invention therefore relates to the use of a veterinary shampoo composition comprising a compound selected from the group of a sphingoid base, a sphingoid base derivative or a mixture of two or more of these compounds, for applying it on non-human mammal during a topical treatment for controlling ectoparasites.

The present invention also relates to a method for controlling ectoparasites in a non-human mammal comprising topically administering to said non-human mammal a veterinary composition comprising an effective amount of one or several active ingredients as insecticides, in combination with a simultaneous or sequential topical administration and rinse of a veterinary shampoo composition comprising a compound selected from the group of a sphingoid base, a sphingoid base derivative or a mixture of two or more of these compounds.

Another object of the present invention is a kit comprising (a) one compartment comprising one or several active ingredients as insecticides and (b) a second compartment comprising a shampoo composition comprising a compound selected from the group of a sphingoid base, a sphingoid base derivative or a mixture of two or more of these compounds, for simultaneous or sequential use, in particular for controlling ectoparasites in a non-human mammal.

### LEGEND TO THE FIGURES

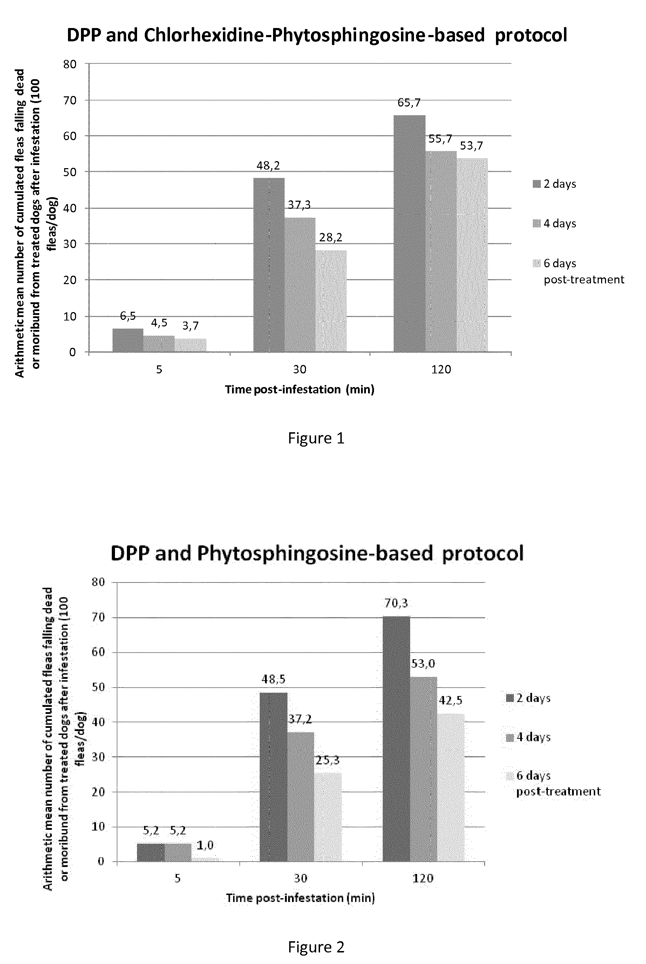

**Figure 1****:** Arythmetic mean number of cumulated fleas falling dead or moribund from treated dogs after infestation (100 fleas/dog) with a dinotefuran-pyriproxyfen-permethrin topical ectoparasiticide treatment and phytosphingosine-based shampoo product (DOUXO^{®}Pyo) application one day after the ectoparasiticide treatment according to the protocol of Example 1.
**Figure 2****:** Arythmetic mean number of cumulated fleas falling dead or moribund from treated dogs after infestation (100 fleas/dog) with a dinotefuran-pyriproxyfen-permethrin topical ectoparasiticide treatment and phytosphingosine-based shampoo product (DOUXO^{®}Calm) application one day after the ectoparasiticide treatment according to the protocol of Example 1.

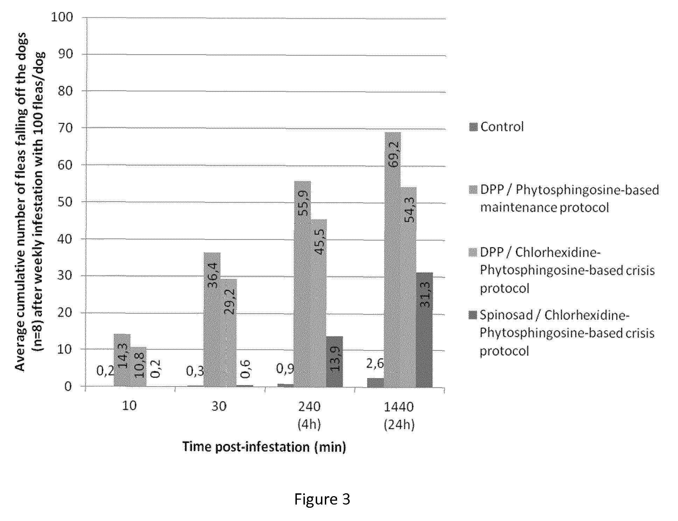

**Figure 3****:** Average cumulative number of fleas falling off the dogs after weekly infestation with 100 fleas/dog with a dinotefuran-pyriproxyfen-permethrin topical ectoparasiticide treatment or a systemic and phytosphingosine-based shampoo product (DOUXO^{®}Calm) application two days after the ectoparasiticide treatment according to the protocol of Example 2.

### DETAILED DESCRIPTION OF THE INVENTION

The type of sphingoid base or sphingoid base derivative used in the shampoo composition of the invention is not critical to the invention.
The sphingoid base is preferably selected from the group of sphingosin, sphinganin and phytosphingosine. Phytosphingosine is more particularly preferred.
Suitable examples of sphingoid base derivatives include N-lactyloyl-phytosphingosine, N-salicyloyl-phytosphingosine, or N-retinoylphytosphingosine, i.e. compounds, which are N-substituted.
Other suitable examples of sphingoid base derivatives include sphingoid base salts. The anion of the salt may be derived from any suitable acid, i.e. those acids, which upon mixing with the sphingoid base in a suitable solid produce a salt with an improved water solubility. The acids which itself are effective when applied to fur or skin are preferred. Preferred salts of the sphingoid base for use in the composition of this invention are the salts obtainable with those acids which upon mixing with the above mentioned sphingoid base in a suitable solvent, produce a salt with an increased water solubility as compared to the water solubility of the sphingoid base as such. The salts of phytosphingosin are preferred for better solubility and better bioavailability.

In one preferred embodiment the acid is a hydrophilic acid capable of delivering the sphingoid base salt to the water phase of the veterinary composition. Suitable hydrophilic acids include a-hydroxy alkanoic acid, a (3-hydroxy alkanoic acid, an a, p-dihydroxy alkanoic acid, an alkanedioic acid or a mineral acid. Examples of preferred hydrophilic organic acids are lactic acid, glycolic acid, malic acid, pyruvic acid, succinic acid, fumaric acid, ascorbic acid, gluconic acid and/or pyroglutamic acid. Examples of preferred mineral acids are hydrochloric acid, nitric acid and/or phosphoric acid.

In another preferred embodiment, the acid is a lipophilic organic acid to allow increasing both the efficacy of the lipophilic acid and the sphingoid base in the sphingoid base salt.

In the shampoo composition of the invention, the concentration of sphingoid base compound is preferably between 0.001 and 20% by weight based on the total weight of the composition. The preferred concentration will usually be adapted to the type of application envisaged. More preferred concentrations may vary from 0.005-10 wt. %, 0.01 to 5 wt. %, 0.02-5 wt. %, or 0.05-3 wt %.

As used herein, the term "shampoo" comprises any composition intended for cleaning fur of non-human mammals and requires rinse with water after applying on the non-human mammal. Generally, shampoos possess certain essential characteristics. Thus a shampoo possesses wetting, foaming and detergent properties in order to ensure satisfactory cleaning of fur. Generally, a shampoo composition of the invention contains surfactants, preferably at least one anionic, cationic, amphoteric, polar non-ionic surface active agent, or a mixture thereof.
Amongst the anionic surfactants, there may be mentioned, in particular: alkyl sulphates (such as magnesium or sodium lauryl sulphates), alkyl ether suIphates (such as lauryl ether sulphate), alkyl polyether sulphates, alkyl sulphonates (the alkyl groups having 8 to 18 carbon atoms), fatty acid soaps, monosulphosuccinates of fatty alcohols, condensation products of fatty adds with isethionic acid, condensation products of fatty acids with methyltaurine, condensation products of fatty adds with sarcosine and condensation products of fatty acids with a protein hydrolysate.

Amongst the cationic surfactants, there may be mentioned in particular: long chain quaternary ammonium compounds, esters of fatty acids and amino-alcohols and polyether amines.
Amongst the polar non-ionic surfactants, there may mentioned in particular: esters of polyols and sugars, condensation products of ethylene oxide with fatty acids, with fatty alcohols, with long chain alkyl-phenols, with long chain mercaptans and with long chain amides, and polyethers of polyhydroxylic fatty alcohols.
Amongst the amphoteric surfactants, there may be mentioned in particular: amino acid derivatives, such as asparagine derivatives, condensation products of monochloroacetic acid with imidazolines and alkylamino-propionates, more specifically betaine or imidazoline derivatives.

In the shampoo compositions, the concentration of surfactants is preferably between 5 and 50% by weight based on the total weight of the composition.

The shampoos according to the invention can also contain other ingredients conventionally used in such compositions, such as viscosity increasing agents, in particular acrylate/methacrylate copolymers, oils, waxes, ceramides, vegetable extracts, emollients, carbomers, gums, in particular xanthan gum, polyethylene glycols, cyclomethicones, conditioners, preservatives, such as methyl para-hydroxybenzoate or propyl parahydroxybeuzoate, dyestuffs, perfumes or a mixture thereof.

The shampoos according to the invention can further comprise active ingredients including disinfectants, such as chlorhexidine.

As used herein, the term "veterinary" denotes for any product or method related to non-human mammals, and more specifically to cats or dogs.

As used herein, "non-human mammals" include any mammals with the exception of humans being. Preferably, non-human mammals are domestic animals including without limitation companion animals or pets such as dogs, cats, rabbits, ferrets and hamsters. In a more preferred embodiment of the invention, non-human mammals are cats and preferably dogs.

According to the invention, the veterinary shampoo composition comprising a compound selected from the group of a sphingoid base, a sphingoid base derivative or a mixture of two or more of these compounds is used for applying it on non-human mammal bodies, preferably skins and fur, during a topical treatment for controlling ectoparasites.

According to another embodiment, the present invention relates to a method for controlling ectoparasites in a non-human mammal comprising topically administering to said non-human mammal a veterinary composition comprising an effective amount of one or several active ingredients as insecticides, in combination with a simultaneous or sequential topical administration and rinse of a veterinary shampoo composition as defined herein comprising a compound selected from the group of a sphingoid base, a sphingoid base derivative or a mixture of two or more of these compounds.

As used herein, the expressions "controlling ectoparasites" and "control of ectoparasites" comprise the reduction, the elimination, the killing, and/or the treatment of ectoparasites in non-human mammals.

As used herein, the term "treatment" includes, particularly, the preventive or curative treatment of non-human mammals against an infestation or infection due to parasites. The preventive treatment of a non-human mammal against a disease designates a treatment made before the non-human mammal has been exposed to or in contact with the causative agent of the infestation (e.g., the ectoparasite), or after said exposure / contact but before development of the symptoms or at an early stage of development of the disease.
In a particular embodiment, the term treatment designates the protection of a non-human mammal against an infestation or infection, e.g., against the effect of an exposure to the causative agent, specifically ectoparasites, or against the development of the disease in exposed-non-human mammals. The invention is particularly suited to protect non-human mammals against an infestation or infection disease such as ectoparasite infestation. The term treatment also includes the alleviation of the symptoms, as well as a delay, reduction or cure of an existing ectoparasite infestation or infection.

The term "ectoparasites" corresponds to a parasite that lives permanently or occasionally on the surface or the skin of a host organism. By way of examples of ectoparasites, arthropod parasites and acari may be cited. Arthropod parasites include without limitation fleas, lice, flies, sand flies and mosquitoes. Acari include without limitation ticks and mites.

The veterinary composition used according to the present invention for the control of ectoparasites can be in any appropriate form for topical administration. Accordingly, the veterinary composition of the invention can be a spot-on, a pour-on, a line-on, a spray, or a dip composition.
In a more preferred embodiment, the composition of the invention is a pour-on, spot-on or a line-on composition.

The "spot-on" (or "drop spot") composition refers to a composition of topical use that is applied to only one spot of the body of the animal where the animal cannot lick the application area and is preferably localized between the animal's shoulders or neck. From this spot, the active ingredients (neonicotinoid compound and N-arylpyrazole compound) spreads rapidly over the entire body surface, thereby providing generalized protection. The active ingredients translocate through the epidermis, accumulates in sebaceous glands, and is gradually released by the follicular ducts.

The "pour-on" or "line-on" composition refer to a composition of topical use that is continuously applied. The "pour-on" compositions are more particularly applied in big non-human mammals such as horses and cattle, and the "line-on" compositions are more particularly applied in small non-human mammals such as domestic animals as defined above, preferably dogs and cats. Particularly, the "pour-on" and "line-on" compositions are applied from the tail, along the backbone to the shoulders of the animal. More particularly, such compositions are applied against the grain of the animal.

The veterinary compositions of the invention are solid, such as powders, gel or liquid forms, and are more preferably a liquid form comprising pharmaceutically acceptable vehicles, such as excipients conventionally used for the preparation of liquid formulations for topical administration in the veterinary field.

Thus, the compositions of the invention may comprise any further excipient currently used in ectoparasiticide compositions such as, without limitation, solvents, antioxidants, binders, fillers, disintegrants, preservatives, crystallization inhibitors, diluents, lubricants, pH modifiers, stabilizers and the like.

The veterinary composition (or the topical treatment) for the control of ectoparasites comprises (or is a topical treatment using) at least one active ingredient as insecticides. The insecticides can be any type of compounds that have the ability to reduce, eliminate, and/or kill ectoparasites. One can cite for instance neonicotinoid compounds, such as dinotefuran, N-arylpyrazole compounds, such as fipronil, or Insect Growth Regulators (IGR), such as pyriproxyfen, chitin inhibitors, macrocyclic lactones, organochlorine or organophosphorus compounds, carbamate compounds, pyrethroid compounds, such as permethrin, semicarbazone compounds, anthelmintic compounds, benzimidazole and imidazothiazole; synergists compounds, and the like.

Neonicotinoid compounds are a class of neuro-active insecticides chemically similar to nicotine. They exhibit an agonist activity for the nicotinic acetylcholine receptors agonists and cause a strong stimulation of nerve cells involving paralysis and death of the parasite with affecting the host animal receptors making them relatively innocuous to the mammals and humans. They have been introduced on market in the 90's and are particularly active against ectoparasites such as fleas, flies and lice. Typical neonicotinoid compounds include without limitation imidacloprid, thiamethoxam, clothianidin, acetamiprid, thiacloprid, dinotefuran, nitenpyram, imidaclothiz, huanyanglin, guadipyr, paichongding, cycloxaprid and the derivatives or salts thereof.

A preferred neonicotinoid compound of the invention is dinotefuran and the derivatives, metabolites or salts thereof. As used in the present description, the term "dinotefuran" can also comprises its derivatives or analogs, metabolites, and salts.

Dinotefuran has been described by the company Mitsui Toatsu Chemicals, Inc. in EP 0 649 845 and has been developed for controlling insect pests. Dinotefuran, also called 2-methyl-1-nitro-3-[(tetrahydro-3-furanyl) methyl] guanidine, has the following formula:

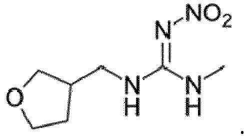

Neonicotinoids and especially dinotefuran are subjected to metabolism in non-human mammals and as a result, metabolites may induce a long-lasting action of neonicotinoids against ectoparasites. The principal metabolic pathways of dinotefuran in mammals involve N-demethylation, nitro reduction, and N-methylene hydroxylation accompanied by amine cleavage. The metabolites of dinotefuran comprise the compounds as disclosed by Simon-Delso et al. (Systemic insecticides (neonicotinoids and fipronil): trends, uses, mode of action and metabolites, Environ. Sci. Pollut. Res., 2015, 22:5-34) and by Ford KA and Casida JE (Unique and common metabolites of thiamethoxam, clothianidin, and dinotefuran in mice, Chem. Res. Toxicol., 2006, 19:1549-1556; Neonicotinoid metabolism: compounds, substituents, pathways, enzymes, organisms, and relevance, J. Agaric. Food Chem., 2011, 59:2923-2931) and FAO dinotefuran (http://www.fao.org/fileadmin/templates/agphome/documents/ PestsPesticides/JMPR/Evaluation12/Dinotefuran.pdf). Typically, the metabolite of dinotefuran comprise, without limitation, N-desmethyl dinotefuran (2-nitro-1-(tetrahydro-3-furylmethyl)guanidine), DIN-NNO, DIN-dm-NNO, DIN-NNH2, 1-methyl-3-(tetrahydro-3-furylmethyl)guanidine, 3-(tetrahydro-3-furylmethyl)guanidine, 1-methyl-3-(tetrahydro-3-fuylmethyl)urea, 3-(tetrahydro-3-fuylmethyl)urea, 2-hydroxy-dinotefuran, 4-hydrox-dinotefuran, 1,3-diazinane aminocarbinol, DIN-b (derivative of DIN-dm), DIN-e (guanidine derivative of DIN-a), DIN-f (guanidine derivative of DIN-b), DIN-g (derivative of DIN-5-OH), DIN-h (desmethyl-g), DIN-I (nitroso derivative of DIN-g), DIN-j (nitrosoderivative of DIN-h), DIN-k (guanidine derivative of DIN-h), tetrahydrofuran carboxaldehyde (3-furfural), tetrahydrofuran alcohol (3-furfuryl alcohol), tetrahydrofuran-3carboxylic acid, 4-hydroxy-tetrahydrofuran-3-carboxylic acid, tetrahydrofuran-3-yl-methylamine, 1-[4-hydroxy-2-(hydroxymethyl)butyl]-3-methyl-2-nitroguanidine, and 3-hydroxy dinotefuran.

Typically, the derivatives or analogs of dinotefuran comprise all the compounds as disclosed in the EP 0 649 845 patent. More particularly, the derivatives or analogs of dinotefuran comprise, without limitation, 1-[{(tetrahydro-3-furanyl)methyl}amino]-1-methylamino-2-nitroethylene, 1-[{(tetrahydro-3-furanyl)methyl}amino]-1-ethylamino-2-nitroethylene, 1-[{(tetrahydro-3-furanyl)methyl}amino]-1-dimethylamino-2-nitroethylene, 1-[{(tetrahydro-3-furanyl)methyl}amino]-1-(1-pyrrolidinyl)-2-nitroethylene, 1-[N-{(tetrahydro-3-furanyl)methyl}-N-methylamino]-1-methylamino-2-nitroethylene, 1-[N-{(tetrahydro-3-furanyl)methyl}-N-propylamino]-1-methylamino-2-nitroethylene, 1-[N-{(tetrahydro-3-furanyl)methyl}-N-propylamino]-1-ethylamino-2-nitroethylene, 1-{(tetrahydro-3-furanyl)methyl}-2-nitro-3-methylguanidine, N-{(tetrahydro-3-furanyl)-methyl}-N-(methyl)nitroguanidine, 1-{(tetrahydro-3-furanyl)methyl}-1-ethyl-2-nitro-3-methylguanidine, N-(tetrahydro-3-furanyl)-methyl-N'-cyano(methylthio)formamidine, N-cyano-N'- {(tetrahydro-3-furanyl)methyl} acetamidine, N-cyano-N'- {(tetrahydro-3-furanyl)methyl}-N-methylacetamidine, N-[4-{(2-methyl)tetrahydrofuranyl}methyl]-N'-methyl-N"-nitroguanidine, 1-{(tetrahydro-3-furanyl)methyl}-1,2-dicyclohexylcarbonyl-2-methyl-3-nitroguanidine, 1-{(tetrahydro-3-furanyl)methyl}-1,2-diethylcarbonyl-2-methyl-3-nitroguanidine, 1-{(tetrahydro-3-furanyl)methyl}-1,2-dimethoxycarbonyl-2-methyl-3-nitroguanidine, and 1-{(tetrahydro-3-furanyl)methyl}-1,2-dibenzoyl-2-methyl-3-nitroguanidine.

N-arylpyrazole compounds are a class of neuro-active insecticides with efficacy against a broad spectrum of tick species. They achieve their efficacy by disrupting the central nervous system by blocking the passage of chloride ions through the GABA (gamma-amiobutyric acid) receptor and glutamate-gated chloride channels (GluCl), components of the central nervous system. This disruption causes hyperexcitation of contamined nerves and muscles, which results to the death of the tick. Typical N-arylpyrazole compounds include without limitation ethiprole, pyriprole, fipronil, sisapronil, butene fipronil and the derivatives or salts thereof.

A preferred N-arylpyrazole compound of the invention is fipronil and the derivatives or salts thereof. As used herein, the term "fipronil" can also comprise its derivatives or analogs, metabolites, and salts.

Fipronil has been discovered at end of the 80's and has been placed on the market under the patent EP 0 295 117. Fipronil, also called (*RS*)-5-amino-1-[2,6-dichloro-4-(trifluoromethyl)phenyl]-4-(trifluoromethylsulfinyl)-1H-pyrazole-3-carbonitrile or fluocyanobenpyrazole has the following formula:

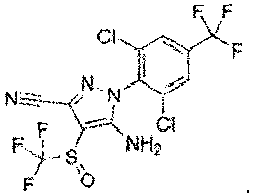

N-arylpyrazole and especially fipronil are subjected to metabolism in non-human mammals and as a result, metabolites may induce a long-lasting action of N-arylpyrazole against ectoparasites. In mammals, fipronil can be metabolized at its trifluoromethylsulfinyl or cyano moieties through three major pathways: (1) oxidation at the sulfinyl moiety to form fipronilsulfone; (2) reduction at the sulfinyl moiety yielding fipronilsulfide; and (3) by hydrolysis of the cyano moiety to form fipronil-amide followed by further hydrolysis to the corresponding carboxylic acid (5-amino-1-(2,6-dichloro-4-trifluoromethylphenyl)-4-trifluoromethylsulfinyl pyrazole-3-carboxylic acid). The metabolites of fipronil comprise the compounds as disclosed by Simon-Delso et al. (Systemic insecticides (neonicotinoids and fipronil): trends, uses, mode of action and metabolites, Environ. Sci. Pollut. Res., 2015, 22:5-34), and in the Draft Assessment Report (DAR) (2005, Initial risk assessment provided by the rapporteur Member State France for the existing active substance fipronil of the second stage of the review programme referred to in Article 8(2) of Council Directive 91/414/EEC*)* and FAO fipronil (http://www.fao.org/fileadmin/templates/agphome/documents/ PestsPesticides/JMPR/Evaluation01/08Fipronil.pdf*).* Typically, the metabolite of fipronil comprise, without limitation, 5-amino-3-cyano-1-(2,6-dichloro-4-trifluoromethylphenyl) pyrazole, 5-amino-1-[2,6-dichloro-4-(trifluoromethyl)phenyl]-4-[(trifluoromethyl)thiol-1H-pyrazole-3-carbonitrile, 5-amino-1-[2,6-dichloro-4-(trifluoromethyl)phenyl]-4-[(trifluoromethyl)sulfonyl]-1H-pyrazole-3-carbonitrile, 5-amino-1-[2,6-dichloro-4-(trifluoromethyl)phenyl]-4-[(1R,S)-(trifluoromethyl)]-1H-pyrazole-3-carbonitrile, 5-amino-3-cyano-1-(2,6-dichloro-4-trifluoromethylphenyl)-pyrazole-4-sulfonic acid, 5-amino-3-carbamyl-1-(2,6-dichloro-4-trifluoromethylphenyl)-4-trifluoromethylsulfonylpyrazole, 5-amino-3-carbamyl-1-(2,6-dichloro-4-trifluoromethylphenyl)-4-trifluoromethylsulfinylpyrazole, 5-amino-3-carbamyl-1-(2,6-dichloro-4-trifluoromethylphenyl)-4-trifluoromethylsulfinylpyrazole-3-carboxylic acid, 5-amino-1-(2,6-dichloro-4-trifluoromethylphenyl)-4-trifluoromethylsulfonylpyrazole-3-carboxylic acid, 1-(2,6-dichloro-4-trifluoromethylphenyl)-3-amino-5-amino-4-trifluoromethylsulfonylpyrazole, 5-amino-3-cyano-1-(2,6-dichloro-4-trifluoromethylphenyl)pyrazole-4-carboxylic acid, 2-[[2,6-dichloro-4-(trifluoromethyl)phenyl]diazenyl]acetonitrile, Ring-opened 5-amino-3-cyano-1-(2,6-dichloro-4-trifluoromethylphenyl)pyrazole-3,4-dicarboxylic acid, and RPA 106681.

Typically, the derivatives or analogs of fipronil comprise all the compounds as disclosed in the EP 0 295 117 patent. More particularly, the derivatives or analogs of fipronil comprise, without limitation, 5-amino-3-cyano-1-(2,6-dichloro-4-trifluoromethylphenyl)-4-trifluoromethylthiopyrazole, 5-amino-3-cyano-1-(2,6-dichloro-4-trifluoromethoxyphenyl)-4-trifluoromethylthiopyrazole, 5-amino-3-cyano-1-(2,6-dichloro-4-difluoromethoxyphenyl)-4-trifluoromethylthiopyrazole, 5-amino-1-(2-chloro-4-trifluoromethylphenyl)-3-cyano-4-trifluoromethylthiopyrazole, 5-amino-3-cyano-1-(2,4,6-trichlorophenyl)-4-trifluoromethylthiopyrazole, 5-amino-3-cyano-1-(2,6-dibromo-4-trifluoromethylphenyl)-4-trifluoromethylthiopyrazole, 5-amino-1-(2-bromo-4-trifluoromethylphenyl)-3-cyano-4-trifluoromethylthiopyrazole, 5-amino-3-cyano-1-(2,6-dichloro-4-trifluoromethylphenyl)-4-difluoromethylthiopyrazole, 5-amino-3-cyano-1-(2,6-dichloro-4-trifluoromethylphenyl)-4-heptafluoropropylthiopyrazole, 5-amino-1-(2-bromo-6-chloro-4-trifluoromethylphenyl)-3-cyano-4-trifluoromethylthiopyrazole, 5-amino-3-cyano-1-(2,6-dichloro-4-trifluoromethylphenyl)-4-trichloromethylthiopyrazole, 5-amino-3-chloro-1-(2,6-dichloro-4-trifluoro-methylphenyl)-4-trifluoromethylthiopyrazole, 5-amino-3-bromo-1-(2,6-dichloro-4-trifluoromethylphenyl)-4-trifluoromethylthiopyrazole, 5-amino-1-(2,6-dichloro-4-trifluoromethylphenyl)-3-fluoro-4-trifluoromethylthiopyrazole, 5-amino-4-chlorodifluoromethylthio-3-cyanol-(2,6-dichloro-4-trifluoromethylphenyl)pyrazole, 5-chloro-3-cyano-1-(2,6-dichloro-4-trifluoromethylphenyl)-4-trifluoromethylthiopyrazole, 5-amino-3-chloro-1-(2,6-dichloro-4-trifluoromethylphenyl)-4-methanesulphonylpyrazole, 3-cyano-1-(2,6-dichloro-4-trifluoromethylphenyl)-5-ethoxymethyleneamino-4-trifluoromethylthiopyrazole, 3-cyano-1-(2,6-dichloro-4-trifluoromethylphenyl)-5-ethoxyethylideneamino-4-trifluoromethylthiopyrazole, 3-cyano-1-(2,6-dichloro-4-trifluoromethylphenyl)-5-ethoxymethyleneamino-4-methanesulphonylpyrazole, 5-acetamido-3-cyano-1-(2,6-dichloro-4-trifluoromethylphenyl)-4-trifluoromethylthiopyrazole, 3-cyano-1-(2,6-dichloro-4-trifluoromethylphenyl)-5-bis(propionyl)amino-4-trifluoromethylthiopyrazole, 3-cyano-1-(2,6-dichloro-4-trifluoromethylphenyl)-5-propionamido-4-trifluoromethylthiopyrazole, 5-acetamido-3-cyano-1-(2,6-dichloro-4-trifluoromethylphenyl)-4-methanesulphonylpyrazole, 3-cyano-1-(2,6-dichloro-4-trifluoromethylphenyl)-4-trifluoromethylthio-5-trimethylacetamidopyrazole, 3-cyano-1-(2,6-dichloro-4-trifluoromethylphenyl)-5-bis(methoxycarbonyl)amino-4-trifluoromethylthiopyrazole, 3-cyano-1-(2,6-dichloro-4-trifluoromethylphenyl)-5-bis(ethoxycarbonyl)amino-4-trifluoromethylthiopyrazole, 5-chloroacetamido-3-cyano-1-(2,6-dichloro4-trifluoromethylphenyl-4-trifluoromethylthiopyrazole, 3-cyano-1-(2,6-dichloro-4-trifluoromethylphenyl)-5-bis(ethoxycarbonyl)amino-4-methanesulphonylpyrazole, 3-cyano-1-(2,6-dichloro-4-trifluoromethylphenyl)-4-methanesulphonyl-5-trimethylacetamidopyrazole, 3-cyano-1-(2,6-dichloro-4-trifluoromethylphenyl)-5-dimethylamino-4-trifluoromethylthiopyrazole, 3-cyano-1-(2,6-dichloro-4-trifluoromethylphenyl)-5-isopropylamino-4-trifluoromethylthiopyrazole, 3-cyano-1-(2,6-dichloro-4-trifluoromethylphenyl)-5-propylamino-4-trifluoromethylthiopyrazole, 3-cyano-1-(2,6-dichloro-4-trifluoromethylphenyl)-5-dipropylamino-4-trifluoromethylthiopyrazole, 3-cyano-1-(2,6-dichloro-4-trifluoromethylphenyl)-5-bis(propargyl)amino-4-trifluoromethylthiopyrazole, 3-cyano-1-(2,6-dichloro-4-trifluoromethylphenyl)-5-methylamino-4-methanesulphonylpyrazole, 5-bromo-3-cyano-1-(2,6-dichloro-4-trifluoroethylphenyl)-4-trifluoromethanesulphonylpyrazole, 5-bromo-3-cyano-1-(2,6-dichloro-4-trifluoromethylphenyl)-4-trifluoromethylthiopyrazole, 5-bromo-3-cyano-1-(2,6-dichloro-4-trifluoromethylphenyl)-4-methanesulphonylpyrazole, 5-amino-3-bromo-1-(2,6-dichloro-4-trifluoromethylphenyl)-4-methanesulphonylpyrazole, 3-bromo-1-(2,6-dichloro-4-trifluoromethylphenyl)-4-methanesulphonylpyrazole, 3-cyano-1-(2,6-dichloro-4-trifluoromethylphenyl)-4-trifluoromethylthiopyrazole, 3-cyano-1-(2,6-dichloro-4-trifluoromethylphenyl)-4-trifluoromethanesulphonylpyrazole, 3-cyano-1-(2,6-dichloro-4-trifluoromethoxyphenyl)-4-trifluoromethylthiopyrazole, 3-cyano-1-(2,6-dichloro-4-trifluoromethylphenyl)-4-methanesulphonylpyrazole, 3-cyano-1-(2,6-dichloro-4-trifluoromethylphenyl)-5-iodo-4-trifluoromethylthiopyrazole, 3-cyano-1-(2,6-dichloro-4-trifluoromethylphenyl)-5-iodo-4-trifluoromethanesulphonylpyrazole, 5-amino-1-(2,6-dichloro-4-trifluoromethylphenyl)-3-iodo-4-methanesulphonylpyrazole, 1-(2,6-dichloro-4-trifluoromethylphenyl)-3-iodo-4-methanesulphonylpyrazole, 1-(2,6-dichloro-4-trifluoromethylphenyl)-3-iodo-4-trifluoromethylthiopyrazole, 5-amino-3-cyano-1-(2,6-dichloro-4-trifluoromethylphenyl)-4-trifluoromethanesulphonylpyrazole, 5-amino-3-cyano-1-(2,6-dichloro-4-trifluoromethylphenyl)-4-trifluoromethylsulphinylpyrazole, 5-amino-3-cyano-1-(2,6-dichloro-4-trifluoromethoxyphenyl)-4-trifluoromethanesulphonylpyrazole, 5-amino-3-cyano-1-(2,6-dichloro-4-trifluoromethoxyphenyl)-4-trifluoromethylsulphinylpyrazole, 3-cyano-1-(2,6-dichloro-4-trifluoromethylphenyl)-4-trifluoromethylsulphinylpyrazole, 5-amino-3-cyano-1-(2,6-dichloro-4-trifluoromethylphenyl)-4(1-methylprop-2-ynylsulphinyl)pyrazole, 5-amino-3-cyano-1-(2,6-dichloro-4-trifluoromethylphenyl)-4-methylsulphinylpyrazole, 5-amino-3-cyano-1-(2,6-dichloro-4-trifluoromethylphenyl)-4-isopropylsulphinylpyrazole, 5-amino-3-bromo-1-(2,6-dichloro-4-trifluoromethylphenyl)-4-trifluoromethylsulphinylpyrazole, 5-amino-4-tert-butanesulphonyl-3-cyano-1-(2,6-dichloro-4-trifluoromethylphenyl)pyrazole, 3-cyano-1-(2,6-dichloro-4-trifluoromethylphenyl)-5-propylamino-4-trifluoromethylsulphonylpyrazole, 5-acetamido-3-cyano-1-(2,6-dichloro-4-trifluoromethylphenyl)-4-trifluoromethanesulphonylpyrazole, 1-(2,6-dichloro-4-trifluoromethylphenyl)-4-methanesulphonyl-3-nitropyrazole, 5-amino-1-(2,6-dichloro-4-trifluoromethylphenyl)-4-methanesulphonyl-3-nitropyrazole, 1-(2,6-dichloro-4-trifluoromethylphenyl)-3-nitro-4-trifluoromethylsulphinylpyrazole, 5-amino-1-(2-bromo-6-chloro-4-trifluoromethylphenyl)-3-cyano-4-methanesulphonylpyrazole, 5-amino-1-(2,6-dichloro-4-trifluoromethoxyphenyl)-3-cyano-4-methanesulphonylpyrazole, 3-acetyl-5-amino-1-(2,6-dichloro-4-trifluoromethylphenyl)-4-methanesulphonylpyrazole, 5-amino-3-cyano-1-(2,6-dichloro-4-trifluoromethylphenyl)-4-methylthiopyrazole, 5-amino-3-cyano-1-(2,6-dichloro-4-trifluoromethylphenyl)-4-ethylthiopyrazole, 5-amino-3-cyano-1-(2,6-dichloro-4-trifluoromethylphenyl)-4-propylthiopyrazole, 5-amino-3-cyano-1-(2,6-dichloro-4-trifluoromethylphenyl)-4-isopropylthiopyrazole, 5-amino-3-cyano-1-(2,6-dichloro-4-trifluoromethylphenyl)-4-(2-methylpropylthio)pyrazole, 5-amino-3-cyano-1-(2,6-dichloro-4-trifluoromethylphenyl)-4-(1-methylpropylthio)pyrazole, 4-allylthio-5-amino-3-cyano-1-(2,6-dichloro-4-trifluoromethylphenyl)pyrazole, 5-amino-3-cyano-1-(2,6-dichloro-4-trifluoromethylphenyl)-4-(prop-2-ynylthio)pyrazole, 5-amino-3-cyano-1-(2,6-dichloro-4-trifluoromethylphenyl)-4-(1-methylprop-2-ynylthio)pyrazole, 5-amino-3-bromo-1-(2,6-dichloro-4-trifluoromethylphenyl)-4-methylthiopyrazole, 5-amino-3-cyano-1-(2,6-dichloro-4-trifluoromethylphenyl)-4-tert-butylthiopyrazole, 5-amino-3-bromo-1-(2,6-dichloro-4-trifluoromethylphenyl)-4-methylsulphinylpyrazole, 5-amino-3-cyano-1-(2,6-dichloro-4-trifluoromethylphenyl)-4-ethanesulphonylpyrazole, 3-cyano-1-(2,6-dichloro-4-trifluoromethylphenyl)-5-methylamino-4-trifluoromethylthiopyrazole, 3-cyano-1-(2,6-dichloro-4-trifluoromethylphenyl)-5-(N-ethoxycarbonyl-N-methyl)amino-4-trifluoromethylthiopyrazole, 3-cyano-1-(2,6-dichloro-4-trifluoromethylphenyl)-5-trifluoroacetamido-4-trifluoromethylthiopyrazole, 3-cyano-1-(2,6-dichloro-4-trifluoromethylphenyl)-5-(ethoxycarbonylamino)-4-trifluoromethylthiopyrazole, 3-acetyl-1-(2,6-dichloro-4-trifluoromethylphenyl)-4-trifluoromethylthiopyrazole, 1-(2,6-dichloro-4-trifluoromethylphenyl)-3-formyl-4-trifluoromethylthiopyrazole, 5-amino-1-(2,6-dichloro-4-trifluoromethylphenyl)-3-formyl-4-trifluoromethylthiopyrazole, 3-cyano-1-(2,6-dichloro-4-trifluoromethylphenyl)-5-fluoro-4-trifluoromethanesulphonylpyrazole, 5-amino-3-cyano-4-dichlorofluoromethylthio-1-(2,6-dichloro-4-trifluoromethylphenyl)pyrazole, 5-amino-3-chloro-1-(2,6-dichloro-4-trifluoromethylphenyl)-4-trifluoromethanesulphonylpyrazole, 5-amino-3-cyano-1-(2,6-dichloro-4-trifluoromethylphenyl)-4-pentafluoroethylthiopyrazole, 3-cyano-1-(2,6-dichloro-4-trifluoromethylphenyl)-5-dimethylamino-4-trifluoromethylsulphinylpyrazole, 3-cyano-1-(2,6-dichloro-4-trifluoromethylphenyl)-5-iodo-4-trifluoromethylsulphinylpyrazole, 5-bromo-3-cyano-1-(2,6-dichloro-4-trifluoro-methylphenyl)-4-trifluoromethylsulphinylpyrazole, 5-acetamido-3-cyano-1-(2,6-dichloro-4-trifluoromethylphenyl)-4-trifluoromethylsulphinylpyrazole, 3-cyano-1-(2,6-dichloro-4-trifluoromethylphenyl)-5-bis(ethoxycarbonyl)amino-4-trifluoromethanesulphonylpyrazole, 3-cyano-1-(2,6,dichloro-4-trifluoromethylphenyl)-5-ethoxycarbonylamino-4-trifluoromethanesulphonylpyrazole, 3-cyano-1-(2,6-dichloro-4-trifluoromethylphenyl)-5-ethoxymethyleneamino-4-trifluoromethanesulphonylpyrazole, 5-amino-4-(2-chloro-1,1,2-trifluoroethylthio)-3-cyano-1-(2,6-dichloro-4-trifluoromethylphenyl)pyrazole, and 3-cyano-1-(2,6-dichloro-4-trifluoromethylphenyl)-5-dimethylamino-4-trifluoromethanesulphonylpyrazole.

Within the context of the invention, the salts of dinotefuran and fipronil correspond to its acceptable pharmaceutically salts. An acceptable pharmaceutically salt includes inorganic as well as organic acids salts. Representative examples of suitable inorganic acids include hydrochloric, hydrobromic, hydroiodic, phosphoric and the like. Representative examples of suitable organic acids include formic, acetic, trichloroacetic, propionic, benzoic, cinnamic, fumaric, maleic, methanesulfonic and the like.

Other pesticides/insecticides include a "growth regulator" (also called "IGR" or "Insect Growth Regulator") such as S-methoprene or methoprene, pyriproxyfen, a chitin inhibitor such as lufenuron, chlorfluazuron, hexaflumuron, cyromazine, and 1-(2,6-difluorobenzoyl)-3-[2-fluoro- 4- (1,1,2,3,3,3-hexafluoropropoxy) phenyl urea]; a macrocyclic lactone such as avermectin, typically ivermectin, abamectin, doramectin, eprinomectin, selamectin, and milbemycins such as, milbemycin oxime, moxidectin and abamectin; a organochlorine or organophosphorus compound such as diazinon, coumaphos, dichlorvos, fenitrothion, fenthion, bendiocarb, tetrachlorvinphos, and chlorpyrifos; a carbamate compound such as propoxur, carbaryl, metoxadiazone and Fenobucarb; a pyrethroid compound such as permethrin, deltamethrin, cypermethrin, phenothrin, allethrin, pyrethrin, cyphenothrin, cyfluthrin, fenvalerate, fenpropathrin and transfluthrin; a semicarbazone compound such as metaflumizone; a formamidine compound such as amitraz; an anthelmintic compound such as pyrantel, praziquantel, benzimidazole and imidazothiazole; synergists compounds such as piperonyl butoxide (PBO), octachlorodipropyl ether (S-421), N- (2-ethylhexyl)-bicyclo- [2.2.1]-hept-5-ene-2,3-dicarboximide, isobornyl thiocyanatoacetate (IBTA) and N- (2-ethylhexyl) -1-isopropyl-4-methylbicyclo [2.2.2] oct-5-ene-2,3-dicarboximide.
In a preferred embodiment, the neonicotinoid compound is dinotefuran, derivatives, metabolites or salts thereof.
In another preferred embodiment the N-arylpyrazole compound is fipronil, derivatives, metabolites or salts thereof.

According to a specific embodiment, the veterinary composition (or the topical treatment) for the control of ectoparasites comprises (or uses) dinotefuran, preferably in association with permethrin and/or pyriproxifen.

Within the context of the invention, the expression "effective amount" means the quantity of the active ingredients as insecticides is capable of causing a sufficient control of ectoparasites as above defined. For instance, a sufficient control of ectoparasites is obtained when at least 50 %, 60 %, preferably 70 %, even more preferably at least 80 % of the ectoparasites are killed. The evaluation of the percentage (%) corresponds to the number of ectoparasites that are eliminated or killed relative to the total number of ectoparasites. For instance, a control of at least 50 % means that at least half of the ectoparasites are eliminated or killed and a control of 100% means that the totality of the ectoparasites are eliminated or killed. Therefore, the amount of insecticides may vary in a large extent and depends on several factors including the selected insecticides.

According to an embodiment of the invention, the veterinary shampoo as defined above is topically applied onto a non-human mammal and rinsed thereafter, within a period of time ranging from 2 days before and 2 days after a topical treatment for controlling ectoparasites, preferably from 1 day before and 2 days after a topical treatment for controlling ectoparasites.
According to another embodiment of the invention, the veterinary shampoo as defined above is topically applied onto a non-human mammal and rinsed thereafter the same day of the topical treatment for controlling ectoparasites or one or two days after the topical treatment for controlling ectoparasites starts.

The present invention also relates to a method for controlling ectoparasites in a non-human mammal comprising topically administering to said non-human mammal (e.g. to the fur and/or skin of said mammal) a veterinary composition comprising insecticides as defined above, in combination with a simultaneous or sequential topical administration and rinse of the veterinary shampoo composition as defined above.

The simultaneous or sequential topical administration and rinse of a veterinary shampoo composition preferably includes that the shampoo is used within a period of time ranging from 2 days before and 2 days after a topical treatment for controlling ectoparasites, preferably from 1 day before and 2 days after a topical treatment for controlling ectoparasites.
According to another embodiment of the invention, the veterinary shampoo as defined above is used the same day of the topical treatment for controlling ectoparasites or one or two days after the topical treatment for controlling ectoparasites.

According to specific embodiments, the shampoo of the invention can be used more than once after the topical treatment for controlling ectoparasites, such as two, three, or four times, for example once a week or every two weeks.

Another object of the present invention is a kit comprising (a) one compartment comprising one or several active ingredients as insecticides and (b) a second compartment comprising a shampoo composition comprising a compound selected from the group of a sphingoid base, a sphingoid base derivative or a mixture of two or more of these compounds, for simultaneous or sequential use, in particular for controlling ectoparasites in a non-human mammal.

A further object of the invention is a kit as defined herein, further comprising a package leaflet or user instruction including the information that said compartment (a) is to be used for controlling ectoparasites, preferably fleas and/or ticks in a non-human mammal, preferably in a dog or a cat, and that said compartment (b) is useful for washing/shampooing the non-human mammal, preferably a dog or a cat.

Further aspects and advantages of the invention will be disclosed in the following experimental section.

### EXAMPLES

### Example 1

This study investigates the action of a dinotefuran-pyriproxyfen-permethrin topical ectoparasiticide (DPP, Vectra^{®}3D sold by Ceva Sante Animale) against adult *Ctenocephalides felis* fleas in dogs despite Shampoo and Mousse applications (DOUXO^{®} sold by Ceva Sante Animale) as soon as 24h after administration. DOUXO^{®} Shampoos contain phytoshingosine.

### Material and methods

The protocol was approved by an ethics committee. Eighteen mixed-breed dogs were allocated to 3 groups: an untreated control group (n=6), and 2 DPP-treated groups treated with either a Phytosphingosine-based (n=6, DOUXO^{®}Calm sold by Ceva Sante Animale) or a Chlorhexidine-Phytosphingosine-based (n=6, DOUXO^{®}Pyo sold by Ceva Sante Animale) topical products according to a crisis protocol. Dogs in the treated groups were administered the minimal recommended dose 0.12mL/kg of DPP on day 0. Shampoo (20-30mL) was applied on day 1 and Mousse (1 pump/2kg) on days 3 and 5. Each dog was infested with 100 adult fleas on days 2, 4, and 6. The fleas falling off the dogs were collected 5, 30 and 120min after each infestation before being removed and counted from dogs 2h after infestation. Fleas were categorized as live, moribund or dead. Comparisons between groups were performed on the flea counts by ANOVA (analysis of variance). Veterinary examinations and clinical assessments were performed throughout the study. The Douxo^{®} Calm and Pyo shampoos are as described below.

Douxo^{®}Calm Shampoo composition (Table 1):

**Table 1**

| **Nature/function of the ingredient and/or (INCI Denomination)** | **Content of the INCI ingredient in g/100ml** |
|---|---|
| Anionic surfactant (SODIUM LAURETH SULFATE) | 4.20 |
| Amphoteric surfactant (COCAMIDOPROPYL BETAINE) | 9.0 |
| Non ionic surfactant (DECYLGLUCOSIDE) | 2.65 |
| Cosurfactant (PEG-7 GLYCERYL COCOATE) | 2.5 |
| Solvent (CYCLOMETHICONE) | 1.0 |
| Texturing agent | 2.0 |
| Conditioners | 0.465 |
| Anionic polymers/stabilizing agent | 0.76 |
| Preservative | 0.7 |
| (PHYTOSPHINGOSINE HYDROCHLORIDE) | 0.05 |
| Phytosterol complex | 0.1 |
| (PHYTOSPHINGOSINE BASE) | 0.005 |
| CERAMIDES | 0.015 |
| (CARBOMER) | 0.003 |
| (SODIUM LAURYOL LACTYLATE) | 0.100 |
| (XANTHAN GUM) | 0.003 |
| (CHOLESTEROL) | 0.005 |
| (PHENOXYETHANOL) | 0.009 |
| (ETHYLHEXYLGLYCERIN) | 0.003 |
| Colloidal oatmeal extracts | 0.501 |
| (BUTYLENE GLYCOL) | 0.032 |
| Synthetic peptides | 0.150 |
| (PHENOXYETHANOL) | 0.018 |
| (POLYQUATERNIUM-51) | 0.015 |
| (PHENOXYETHANOL) | 0.0025 |
| pH regulator | 0.16 |
| (AQUA) | QS 100 |

Douxo^{®}Pyo Shampoo composition (Table 2):

**Table 2**

| **Nature/function of the ingredient and/or (INCI Denomination)** | **Content of the INCI ingredient in g/100 ml FP** |
|---|---|
| Amphoteric surfactant (COCAMIDOPROPYL HYDROXY SULTAINE) | 12.0 |
| Lipoaminoacid (CAPRILOYL GLYCINE) | 2.5 |
| Non ionic surfactant (DECYL GLUCOSIDE) | 5.0 |
| PHYTOSPHINGOSINE SALICYLOYL | 0.05 |
| pH regulators | 0.93 |
| Non ionic thickener | 2.0 |
| CHLORHEXIDINE DIGLUCONATE | 3.0 |
| Solvent (PROPYLENE GLYCOL) | 1.00 |
| CLIMBAZOLE | 0.5 |
| FRAGRANCE | 0.1 |
| Preservatives | 0.51 |
| AQUA | QS 100 |

### Results and conclusion

All topical administrations were well tolerated. In average, control dogs retained 84 fleas, with less than 2 fleas falling off. In the treated dogs, there was in average >37 fleas in 30min and >55 fleas knock-down in 2h. The insecticidal efficacy in 2h of DPP was >90% only 2 days after administration and despite DOUXO^{®} Shampoo application 24h after administration. This study confirmed the strong knock-down and speed of kill performances of DPP against fleas despite DOUXO^{®} Shampoo and Mousse procedures.

### Example 2

This study compares the insecticidal and knock-down actions of a dinotefuran-pyriproxyfen-permethrin topical ectoparasiticide (DPP, Vectra^{®}3D) and a spinosad tablet (S, Comfortis^{®}) against adult *Ctenocephalides felis* fleas in dogs, despite Shampoo and Mousse applications (DOUXO^{®}) over one month.

### Material and methods

An ethics committee approved the protocol. Thirty-two mixed-bred dogs were allocated to 4 groups: an untreated control group (n=8), an oral S-treated group receiving Chlorhexidine-Phytosphingosine-based products (n=8, DOUXO^{®}Pyo sold by Ceva Sante Animale) according to crisis protocol and 2 DPP-treated groups receiving either Chlorhexidine-Phytosphingosine-based products (n=8, DOUXO^{®}Pyo) according to crisis protocol or Phytosphingosine-based products (n=8, DOUXO^{®}Calm sold by Ceva Sante Animale) according to maintenance protocol. Dogs in the treated groups were administered the European label dose of ectoparasiticides on day 0. Chlorhexidine-Phytosphingosine-based crisis protocol consisted of applying Shampoo (20-30mL) on days 2, 9, 16, 23 and Mousse (1 pump/2kg) on days 5, 7, 12, 14,19,21. Phytosphingosine-based maintenance protocol consisted of applying Shampoo (20-30mL) on day 2 and Mousse (1 pump/2kg) on days 9, 16, 23. Each dog was infested with 100 adult fleas on days - 6, - 4, 3, 10, 17, 24, and 29. The fleas falling off the dogs were collected 10, 30, 240 min and 24 h after each infestation, for knock-down assessment; before being removed and counted from dogs 24h after infestation for insecticidal assessment. Fleas were categorized as live, moribund or dead. Comparisons between groups were performed on the flea counts by ANOVA. Veterinary examinations and clinical assessments were performed throughout the study. The Douxo^{®} Calm and Pyo shampoos are as described above in example 1.

### Results and conclusion

All products were well tolerated. In average, control dogs retained 74 fleas, with less than 3 fleas falling off. Whatever the procedure, more fleas were dislodged in average from the DPP-treated than from the systemic S-treated dogs. This study demonstrates that the flea knock-down performances of topical DPP are higher than S despite DOUXO^{®} Shampoo application 48 h and DOUXO^{®} protocols over 1 month.

## Claims

1. A use of a veterinary shampoo composition comprising a compound selected from the group of a sphingoid base, a sphingoid base derivative or a mixture of two or more of these compounds, for applying it on non-human mammal during a topical treatment for controlling ectoparasites.

2. The use according to claim 1, wherein the sphingoid base is selected from the group of sphingosin, sphinganin and phytosphingosine, and is preferably phytosphingosine.

3. The use according to claim 1, wherein the sphingoid base derivative is selected from the group of N-lactyloyl-phytosphingosine, N-salicyloyl-phytosphingosine, or N-retinoylphytosphingosine.

4. The use to any one of claims 1 to 3, wherein the concentration of sphingoid base compound is preferably between 0.001 and 20% by weight based on the total weight of the composition.

5. The use according to any one of claims 1 to 4, wherein the shampoo composition contains surfactants, preferably at least one anionic, cationic, amphoteric, polar non-ionic surface active agent, or a mixture thereof.

6. The use according to any one of claims 1 to 5, wherein the shampoo further comprises active ingredients, preferably at least one disinfectant, and more preferably chlorhexidine.

7. The use according to any one of claims 1 to 6, wherein the topical treatment for controlling ectoparasites is a topical treatment against fleas and/or ticks.

8. The use according to any one of claims 1 to 6, wherein the topical treatment for controlling ectoparasites is a topical treatment using at least one neonicotinoid compound, such as dinotefuran, N-arylpyrazole compound, such as fipronil, or Insect Growth Regulator (IGR), such as pyriproxyfen, chitin inhibitor, macrocyclic lactone, organochlorine or organophosphorus compound, carbamate compound, pyrethroid compound, such as permethrin, semicarbazone compound, anthelmintic compound, benzimidazole and imidazothiazole; synergists compounds, or a mixture thereof.

9. The use according to any one of claims 1 to 8, wherein the non-human mammal is a dog or a cat.

10. The use according to any one of claims 1 to 9, wherein the veterinary shampoo is topically applied onto a non-human mammal and rinsed thereafter, within a period of time ranging from 2 days before and 2 days after a topical treatment for controlling ectoparasites, preferably from 1 day before and 2 days after a topical treatment for controlling ectoparasites.

11. The use according to any one of claims 1 to 10, wherein the veterinary shampoo is topically applied onto a non-human mammal and rinsed thereafter the same day of the topical treatment for controlling ectoparasites or one or two days after starting the topical treatment for controlling ectoparasites.

12. A method for controlling ectoparasites in a non-human mammal comprising topically administering to said non-human mammal a veterinary composition comprising an effective amount of one or several active ingredients as insecticides, in combination with a simultaneous or sequential topical administration and rinse of a veterinary shampoo composition as defined herein comprising a compound selected from the group of a sphingoid base, a sphingoid base derivative or a mixture of two or more of these compounds.

13. The method according to claim 12, wherein the shampoo is used within a period of time ranging from 2 days before and 2 days after a topical treatment for controlling ectoparasites, preferably from 1 day before and 2 days after a topical treatment for controlling ectoparasites.

14. The method according to claim 12 or 13, wherein the veterinary shampoo is used the same day of the topical treatment for controlling ectoparasites or one or two days after the topical treatment for controlling ectoparasites.

15. A kit comprising (a) one compartment comprising one or several active ingredients as insecticides and (b) a second compartment comprising a shampoo composition comprising a compound selected from the group of a sphingoid base, a sphingoid base derivative or a mixture of two or more of these compounds, for simultaneous or sequential use, in particular for controlling ectoparasites in a non-human mammal.
